# EUROPÄISCHE PATENTSCHRIFT

(11) **EP 1 143 910 B1**
(45) Veröffentlichungstag und Bekanntmachung des Hinweises auf die Patenterteilung: **16.03.2005**
(21) Anmeldenummer: 00901564.5
(22) Anmeldetag: 14.01.2000
(51) Int. Cl.: A61J 3/10, B30B 11/16

(54) **VERFAHREN UND VORRICHTUNG ZUR HERSTELLUNG UNTERSCHIEDLICHER FESTER DOSIERUNGSFORMEN**
METHOD AND DEVICE FOR PRODUCING DIFFERENT SOLID DOSAGE FORMS
PROCEDE ET DISPOSITIF POUR PRODUIRE DIFFERENTES FORMES GALENIQUES SOLIDES

(30) Priorität: 15.01.1999 DE 19901383
(43) Veröffentlichungstag der Anmeldung: 17.10.2001
(60) Teilanmeldung: 04016554.0
(73) Patentinhaber: Abbott GmbH & Co. KG, 65205 Wiesbaden (DE)
(72) Erfinder: ROSENBERG, Jörg, D-67158 Ellerstadt (DE); MAIER, Werner, D-67105 Schifferstadt (DE)
(74) Vertreter: Kinzebach, Werner, Dr.
(86) Internationale Anmeldenummer: PCT/EP2000/000270
(87) Internationale Veröffentlichungsnummer: WO 2000/041668

(56) Entgegenhaltungen:
- EP-A- 0 358 105
- DE-A- 19 539 359
- US-A- 5 135 113
- US-A- 5 761 886

## Beschreibung

Die vorliegende Erfindung betrifft eine Vorrichtung und ein Verfahren zur Herstellung fester Dosierungsformen durch Formung eines plastischen wirkstoffhaltigen Gemischs unter Verwendung zweier Formwalzen, die zur Formung des plastischen Gemischs zusammenwirken und eine Vielzahl von Vertiefungen zur Aufnahme und Formung des plastischen Gemischs aufweisen.

Die Herstellung von festen Dosierungsformen, insbesondere pharmazeutischen Dosierungsformen oder Nahrungsmitteln oder Nahrungsergänzungsmitteln für Mensch und Tier, durch Kalandrierung einer wirkstoffhaltigen Schmelze ist aus einer Reihe von Publikationen bekannt. Grundlage dieses Verfahrens ist die Einbettung eines Wirkstoffes in eine Schmelze aus einem Träger, z.B. Fettsubstanzen oder physiologisch verträglichen Polymeren. In der Regel wird dazu in einem Mischer und/oder Extruder eine wirkstoffhaltige Schmelze oder ein Wirkstoff haltiges plastisches Gemisch erzeugt und anschliessend in ein Formwerkzeug, z.B. einen Kalander mit Formwalzen, eingespeist. Der Kalander umfasst ein sich gegenläufig drehendes Formwalzenpaar, wobei die Formwalzen auf ihrer Oberfläche Gravuren (Vertiefungen) aufweisen, die z.B. der Form einer Tablettenhälfte entsprechen. Die Tablettenformung erfolgt im Berührungsbereich der beiden Walzen durch Kombination der Tablettenmasse einer Vertiefung auf der einen Walze mit derjenigen der gegenüberliegenden Vertiefung auf der anderen Walze. Auch die Kombination aus einer Formwalze mit Vertiefungen und einer Glattwalze oder einem glatten Band zur Formung solcher plastischen Gemische ist denkbar. Die Herstellung von Tabletten nach diesem Verfahren beschreiben allgemein die DE-A-17 66 546 und die US-A-4,880,585; die DE-A-44 46 467 beschreibt die Herstellung linsenförmiger Tabletten und die WO-96/19962 die Herstellung von teilbaren Tabletten. Diese Verfahren weisen gegenüber der herkömmlichen Herstellung von Dosierungsformen, durch z.B. Tablettieren von Pulvern und Granulaten unter Druck, erhebliche Vorteile auf. So vereint die Schmelzextrusion mit anschliessender Formung durch Kalandrieren eine Vielzahl von Stufen, wie Zudosieren, Mischen, Plastifizieren, Formen und Vereinzeln, in einem einzigen kontinuierlichen Verfahren und ermöglicht so hohen Durchsatz, gleichbleibende Qualität, weite Freiheit bei der Formgestaltung, stellt geringe Anforderungen an die Konfektionierung der Edukte und ermöglicht so die wirtschaftliche Herstellung hoher Stückzahlen. Diese Vorteile kommen aufgrund der vielen optimierungsbedürftigen Parameter bei so vielen Stufen erst bei wirklich hohen Stückzahlen voll zum Tragen.

In vielen Teilbereichen des Pharmamarktes ist ein Trend zur Diversifizierung bezüglich der Dosierung und der Darreichungsform von Wirkstoffen zu beobachten. Bedingt wird diese Diversifizierung unter anderem durch steigende Ansprüche an die Uniformität der Wirkstoffgabe, die durch teilbare Tabletten in der Regel nicht gewährleistet ist. Die Bereitstellung einer Vielzahl von Dosierungen des gleichen Wirkstoffes wird beispielsweise bei der Einstellung von Patienten auf bestimmte Wirkstoffe, z.B. bei Herz-Kreislauf-Erkrankungen, bei unterschiedlichen Dosisanforderungen/Ansprechzeiten unterschiedlicher Patientengruppen, z.B. Erwachsene/Kinder, benötigt. Auch bei Wirkstoffen mit geringer therapeutischer Breite und bei Wirkstoffen mit mehreren medizinischen Indikationen werden eine grosse Anzahl von Dosierungsformen benötigt, die sich in der Wirkstoffdosis unterscheiden. So liegt beispielsweise bei dem Wirkstoff Acetylsalicylsäure die Dosis bei der Verwendung in Schmerztabletten bei 500 mg, während derselbe Wirkstoff als Thrombozytenaggregationshemmer in einer Dosis von nur 100 mg verabreicht wird. Auch ist es in vielen Fällen wünschenswert, von einer Wirkstoffzubereitung mehrere Ausgestaltungen anbieten zu können, z.B. teilbare oder nicht-teilbare, linsenförmige oder Oblongtabletten, um den Anforderungen verschiedener Märkte oder der Auftragsfertigung zu genügen.

Die Fertigung einer solchen Vielzahl unterschiedlicher Dosierungsformen war bisher nur durch konventionelle Tablettierung möglich, mit den bekannten Nachteilen. Ausserdem lassen sich bei der konventionellen Tablettierung unterschiedliche Dosierungen nur durch unterschiedliche, einzeln anzufertigende Tablettenrezepturen verwirklichen, die simultane Herstellung verschiedener Dosierungen/Formen auf einer Tablettiermaschine ist bisher nicht bekannt und auch nur unter grossen Problemen vorstellbar.

Die US 5,761,886 offenbart ein Verfahren zur Kapselherstellung. Das Füllgut wird zwischen zwei Gelatinefolien eingebracht, die in den Spalt gegenläufig rotierender Formwalzen geführt werden. Die Formwalzen haben Vertiefungen auf ihren Oberflächen. Figur 16 zeigt eine Formwalze mit Vertiefungen unterschiedlicher Gestalt.

Der vorliegenden Erfindung lag daher die Aufgabe zugrunde, ein Verfahren und eine Vorrichtung bereitzustellen, die eine schnelle, effektive und kostengünstige gleichzeitige Herstellung einer Vielzahl unterschiedlicher Dosierungen und/oder Ausformungen eines Wirkstoffs ermöglicht.

Gegenstand der vorliegenden Erfindung ist eine Vorrichtung nach Anspruch 1 und ein Verfahren zur Herstellung fester Dosierungsformen durch Formung eines plastischen wirkstoffhaltigen Gemischs unter Verwendung der Vorrichtung.

### Beschreibung der Figuren

- Figur 1: zeigt einen Längsschnitt durch ein Walzenpaar W1 und W2 mit unterschiedlichen Vertiefungen V1, V1T, V2, V3, V4 und V5.
- Figur 2: zeigt einen Längsschnitt durch ein Walzenpaar W1' und W2.
- Figur 3: zeigt einen Ausschnitt aus der Abrollung der im Beispiel verwendeten Formwalzen. Die Gruppen von Vertiefungen sind in den Spuren 1 bis 8 angeordnet.

Der Begriff "Dosierungsform" bezeichnet vorliegend eine beliebige Darreichungsform zur Verabreichung von Wirkstoffen an Mensch, Tier oder Pflanze. Die erfindungsgemäss erhaltenen Dosierungsformen sind insbesondere zur oralen oder rektalen Verabreichung oder als implantierbare Wirkstoffdepots bei Mensch und Tier geeignet. Besonders bevorzugte Dosierungsformen sind Tabletten jeglicher Form, Dragees, Pellets und Zäpfchen.

Nach dem erfindungsgemässen Verfahren können in einem Arbeitsgang mehrere unterschiedliche Dosierungsformen erhalten werden. Unterschiedliche Dosierungsformen sind Dosierungsformen, die sich im Volumen (und damit in der Wirkstoffdosierung) und/oder in der Formgestaltung (z.B. linsenförmig, länglich, rund; teilbar, nicht-teilbar etc.) unterscheiden.

Während bei dem konventionellen Tablettieren mittels einer Tablettiermaschine die Dichte und damit das Volumen der Tablette nicht nur von der verwendeten Form, sondern auch von der Presskraft abhängt, ist bei der Formung von Dosierungsformen mittels kontinuierlichen Formwerkzeugen, wie z.B. Kalandern mit Formbändern oder -walzen, die einen definierten Abstand aufweisen oder sich berühren, das Volumen der Dosierungsform direkt vom Volumen der Vertiefungen des Formwerkzeugs abhängig. Daher hängt bei diesem Verfahren bei gegebener Rezeptur (d.h. festgelegtem prozentualem Wirkstoffgehalt) der Wirkstoffgehalt der Dosierungsform direkt vom Volumen der Vertiefungen in den Formwerkzeugen ab.
Dies ist möglich, da bei dem erfindungsgemässen Verfahren ein plastisches wirkstoffhaltiges Gemisch verwendet wird, das vorzugsweise im Wesentlichen nicht komprimierbar ist und dessen Dichte daher, im Gegensatz zu den bei der konventionellen Tablettierung eingesetzten Granulaten und Pulvern, in engen Grenzen konstant gehalten werden kann. Daher können mit dem hier beschriebenen Verfahren durch Verwendung von kontinuierlichen Formwerkzeugen mit zwei zusammenwirkenden Teilen, wovon zumindest ein Teil Vertiefungen mit unterschiedlichem Volumen und/oder unterschiedliche Formgebung aufweist, aus einer Rezeptur in einem Arbeitsschritt Dosierungsformen mit unterschiedlichen Wirkstoffdosierungen und/oder unterschiedlicher Form hergestellt werden. Dazu wird das Formwerkzeug im erfindungsgemässen Verfahren mit einem plastischen wirkstoffhaltigen Gemisch möglichst gleichbleibender Zusammensetzung gespeist. Die unterschiedlichen Dosierungsformen werden allein aufgrund der unterschiedlichen Gruppen von Vertiefungen des Formwerkzeugs gebildet beziehungsweise geformt.

Für die Herstellung des plastischen Gemischs ist es erforderlich, die Bestandteile, nämlich mindestens ein thermoplastisches, physiologisch verträgliches, in der Regel polymeres, Bindemittel und mindestens einen Wirkstoff und gegebenenfalls übliche Additive zu vermischen und in ein plastisches Gemisch überzuführen, vorzugsweise in Abwesenheit eines Lösungsmittels. Die Bildung des plastischen Gemischs kann durch Aufschmelzen oder auch durch Kneten, Vermischen oder Homogenisieren unterhalb der Schmelztemperatur des Bindemittels durchgeführt werden. Diese Verfahrensschritte können auf an sich bekannte Art und Weise durchgeführt werden, beispielsweise wie in der EP-A-0 240 904, EP-A-0 337 256, EP-A-0 358 105, WO 97/15290 und WO 97/15291 beschrieben. Auf den Inhalt dieser Publikationen wird hiermit Bezug genommen.

Die Komponenten können zuerst vermischt und dann in den plastischen Zustand überführt und homogenisiert werden. Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, in den plastischen Zustand überzuführen und vorzuvermischen, wobei die Apparaturen, wie Rührkessel, Rührwerke, Feststoffmischer etc., gegebenenfalls im Wechsel betrieben werden, und dann den (die) empfindlichen Wirkstoff(e) in "Intensivmischern" in plastischer Phase bei sehr kleinen Verweilzeiten einzumischen (homogenisieren). Der (die) Wirkstoff(e) kann (können) in fester Form oder als Lösung oder Dispersion eingesetzt werden.

Das Plastifizieren und Vermischen erfolgt in einer für diesen Zweck üblichen Vorrichtung. Besonders geeignet sind Extruder oder beheizbare Behälter mit Rührwerk, z.B. Kneter, (wie der unten noch erwähnten Art).

Als Mischapparat sind auch solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z.B. ORP, CRP, AP, DTB der Firma List oder Reactotherm der Firma Krauss-Maffei oder Ko-Kneter der Fa. Buss), Doppelmulden-kneter (Trogmischer) und Stempelkneter (Innenmischer) oder Rotor/Stator-Systeme (z.B. Dispax der Firma IKA).

Bei empfindlichen wirkstoffen erfolgt vorzugsweise zunächst das Überführen des polymeren Bindemittels in den plastischen Zustand in einem Extruder und anschliessend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z.B. über eine Differentialdosierwaage eingeführt werden. Plastische Massen können direkt aus einem Extruder eingespeist oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Das durch Vermischen und Überführen des Bindemittels, gegebenenfalls des Wirkstoffes und gegebenenfalls des Additivs oder der Additive, in den plastischen Zustand erhaltene Gemisch ist teigig bis zähflüssig (thermoplastisch) und daher auch extrudierbar. Das Bindemittel soll vorzugsweise in physiologischer Umgebung löslich oder quellbar sein. Beispiele für geeignete Bindemittel sind:
Polyvinyllactame, insbesondere Polyvinylpyrrolidon (PVP), Copolymere von Vinyllactamen, wie N-Vinylpyrrolidon, N-Vinylpiperidon und N-Vinyl-E-caprolactam, aber insbesondere N-Vinylpyrrolidon, mit (Meth)acrylsäure, (Meth)acrylsäureestern, Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate, Copolymerisate von Dimethylaminoethylacrylaten und Methacrylestern (z.B. Eudragit-Typen), Polyalkylenglykole, wie Polypropylenglykole und Polyethylenglykole (z.B. Polyethylenglykol 6000), Copolymerisate von Methylmethacrylat und Acrylsäure, Celluloseester, Celluloseether, insbesondere Methylcellulose und Ethylcellulose, Hydroxyalkylcellulosen, insbesondere Hydroxypropylcellulose oder Hydroxypropylmethylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane.

Brauchbar sind auch Gelatine und bioabbaubare Polymere, wie Polyhydroxyalkanoate, z.B. Polyhydroxybuttersäure, Polymilchsäure, Polyaminosäuren, z.B. Polylysin, Polyasparagin, Polydioxane u. Polypeptide.

Bevorzugte polymere Bindemittel sind Polyvinylpyrrolidon, Copolymerisate von N-Vinyllactamen, insbesondere N-Vinylpyrrolidon, und Vinylestern, Copolymerisate von N-Vinyllactamen, insbesondere N-Vinylpyrrolidon, mit (Meth)acrylsäureestern, Polyhydroxyalkylacrylate, Polyhydroxyalkylmethacrylate, Polyacrylate, Polymethacrylate, Alkylcellulosen und Hydroxyalkylcellulosen, insbesondere Hydroxypropylcellulose und Hydroxypropylmethylcellulose.

Vorteilhaft für die Verwendung als plastisches Bindemittel, sind solche Bindemittel, die einen K-Wert (nach H. Fikentscher, Cellulose-Chemie 13 (1932), S. 58-64 und 71-74) im Bereich zwischen 10 und 100, insbesondere zwischen 20 und 80 aufweisen.

Das polymere Bindemittel muss sich in der Gesamtmischung aller Komponenten im Bereich von 50 bis 160°C, vorzugsweise 60 bis 130°C in einen plastischen Zustand überführen lassen. Die Glasübergangstemperatur der Mischung muss daher unter 180°C, vorzugsweise unter 150°C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:

langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-% bezogen auf das Polymerisat, betragen kann, sind z.B. Streckmittel bzw. Füllstoffe, wie Silikate oder Kieselerde, Magnesiumoxid, Aluminiumoxid, Titanoxid, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches;

Schmiermittel und Trennmittel wie Magnesium-, Aluminium- und Calciumstearat, Talkum und Silicone, sowie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Schmier- und Trennmitteln beträgt vorzugsweise 0,1 bis 5 Gew.-%, bezogen auf das Gesamtgewicht der Masse für die jeweilige Schicht; _

Fliessmittel, z.B, Aerosil, in einer Menge von 0,1 bis 5 Gew.-%, bezogen auf das Gesamtgewicht des Gemisches;

Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions- und Formentrennmittel zugesetzt werden (vgl. z.B. H. Sucker et al. Pharmazeutische Technologie, Thieme-Verlag, Stuttgart 1978).

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrit-tetraacetat, Polymere wie z.B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z.B. bei J. L. Ford, Pharm. Acta Helv. 61, 69-88 (1986) angegeben.

Als pharmazeutische Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Voraussetzungen für die Eignung von Hilfsstoffen sind ausreichende Temperaturstabilität und Kompatibilität mit dem verwendeten Wirkstoff.

Unter Wirkstoffen im Sinne der Erfindung sind alle Stoffe mit einer gewünschten Wirkung, insbesondere pharmazeutischen Wirkung auf den menschlichen, tierischen oder pflanzlichen Organismus und möglichst geringen Nebenwirkungen zu verstehen, sofern sie sich unter den Verarbeitungsbedingungen nicht wesentlich zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,001 bis 95, vorzugsweise von 20 bis 80, insbesondere 30 bis 70 Gew.-% liegen. Auch wirkstoff-Kombinationen können eingesetzt werden. Wirkstoffe im Sinne der Erfindung sind auch Vitamine und Mineralstoffe, sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z.B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika und Impfstoffe.

Das erfindungsgemässe Verfahren ist beispielsweise zur Verarbeitung folgender Wirkstoffe bzw. der pharmakologisch aktiven Salze davon geeignet:
Acebutolol, Acetylcystein, Acetylsalicylsäure, Acyclovir, Alfacalcidol, Allantoin, Allopurinol, Alprazolam, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefadroxil, Cefalexin, Cefazolin, Cefixim, Cefotaxim, Ceftazidim, Ceftriaxon, Cefuroxim, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Folinsäure, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Imipramin, Indomethacin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, pantothensäure, Paracetamol, Penicillin G, Penicillin V, Pentoxifyllin, phenobarbital, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Selegilin, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin, Captopril, Omeprazol, Ranitidin, Tramadol, Cyclosporin, Trandolapril und Peptidtherapeutika.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Bei der Extrusion des plastischen Gemisches werden Temperatur, Viskosität und Extrusionsgeschwindigkeit vorteilhaft so gewählt, dass ein zusammenhängendes, selbsttragendes Extrudat erhalten wird. In der Regel wird so kontinuierlich ein Strangextrudat mit vorzugsweise konstantem Querschnitt erzeugt. In vielen Fällen hat es sich als vorteilhaft herausgestellt, schräg abwärts zu extrudieren und/oder gegebenenfalls eine Führungsrinne zum Transport des Extrudats vorzusehen, um einen sicheren Transport zu gewährleisten und ein Abreissen des extrudierten Stranges zu verhindern. In Abhängigkeit von der Anzahl und Verträglichkeit der einzusetzenden Wirkstoffe können vorteilhaft auch mehrschichtige Extrudate, z.B. Coextrudate, wie in der WO 96/19963 beschrieben, bei dem erfindungsgemässen Verfahren eingesetzt werden.

Das vorstehend beschriebene plastische Wirkstoff haltige Gemisch wird der erfindungsgemässen Vorrichtung zugeführt.

Unter Vertiefungen sind hier Aussparungen oder Gravuren an der Oberfläche der Walzen zur Aufnahme und Formung des plastischen wirkstoffhaltigen Gemischs zu verstehen. Die äussere Begrenzung der Vertiefung an der Oberfläche des jeweiligen Teils wird als Umrisslinie bezeichnet. Damit die Dosierungsformen nach der Formung aus den Vertiefungen entnommen werden können, weisen die Vertiefungen in der Regel an der Oberfläche des jeweiligen Teils, z.B. der Mantelfläche einer Formwalze, den grössten Querschnitt auf. Gegebenenfalls ist hinter dem Formwerkzeug noch eine Abstreifvorrichtung, z.B. eine Abstreifwalze, Abstreifbürste o. ä., angebracht, die die Entnahme der Dosierungsformen ermöglicht oder erleichtert und/oder das Formwerkzeug, insbesondere dessen Teile mit Vertiefungen, reinigt.

Im erfindungsgemässen Verfahren setzt man zwei Formwalzen ein, wobei die Formwalzen gegenläufig rotieren und die Umrisslinien der Vertiefungen der einen Formwalze mit den Umrisslinien der Vertiefungen der anderen Formwalze im Spalt zwischen den Formwalzen paarweise im Wesentlichen zur Deckung kommen.

Als Spalt wird hier der Bereich bezeichnet, in dem sich die zwei Teile des Formwerkzeugs am nächsten kommen, unabhängig davon, ob sich die Oberflächen (z.B. Mantelflächen) der Teile berühren oder ob diese beabstandet sind.

Vorzugsweise sind solche gegenläufigen Formwalzen in einem Kalander angeordnet. Das plastische Gemisch kann z.B. mittels eines Füllkeils in den von den beiden Formwalzen gebildeten trogähnlichen Zwischenraum gefüllt werden. Das plastische Gemisch wird dann von den Vertiefungen der gegenläufig rotierenden Formwalzen aufgenommen und geformt. Vorzugsweise wird der Abstand der beiden Formwalzen so gewählt, dass die Oberflächen der Formwalzen einen Spalt mit geringer Dicke bilden, vorzugsweise kleiner als 5 mm und insbesondere kleiner als 1 mm, oder sich die Oberflächen der Formwalzen berühren. Wenn man Dosierungsformen erhalten möchte, die möglichst wenig überstehende Pressnähte oder Grate aufweisen, ist es vorteilhaft, einen möglichst geringen Abstand der Teile des Formwerkzeugs, z.B. der Formwalzen, einzustellen, damit ein möglichst dünner Spalt resultiert. Wenn man die Dosierungsformen als zusammenhängende Streifen von Dosierungsformen erhalten möchte, kann es vorteilhaft sein, den Abstand der Teile des Formwerkzeugs und damit den Spalt etwas dicker zu wählen oder in Umfangsrichtung zwischen den Vertiefungen auf der Mantelfläche der Formwalzen einen Verbindungskanal vorzusehen, der sich bei der Formung der Dosierungsform ebenfalls mit dem plastischen Gemisch füllt und so benachbarte Dosierungsformen mit Verbindungsgraten oder -stegen zu Streifen ("Perlenschnur") oder zu Bändern mit mehreren Reihen miteinander verbundener Dosierungsformen verbindet.

Erfindungsgemäss sind die Vertiefungen der Walzen mindestens zwei Gruppen zuzuordnen wobei sich die Vertiefungen unterschiedlicher Gruppen im Volumen und/oder der Form unterscheiden. In der Regel beträgt die Zahl der Vertiefungen mehr als fünf, insbesondere mehr als zehn oder mehr als fünfzig. Es ist unerheblich, wieviel Vertiefungen zu der jeweiligen Gruppe gehören und wie die Vertiefungen einer Gruppe angeordnet sind.

Unterschiedliche Gruppen von Vertiefungen können sich in der Gestaltung der Umrisslinien unterscheiden, z.B. länglich, elliptisch oder rund, in der Dimension der Umrisslinien, z.B. gross oder klein, und/oder in der Ausgestaltung der Vertiefungen selbst, z.B. im Volumen, in den Krümmungsradien der begrenzenden Flächen der Vertiefungen, z.B. linsenartig flach, halbrund, halbkugelig und/oder durch das Vorliegen von Stegen, die zur Bildung von Bruchrillen in den Dosierungsformen und damit zu teilbaren Dosierungsformen führen.

Bei Verwendung von zwei Formwalzen kann eine Formwalze W1 mit einheitlichen Vertiefungen V1 mit einer zweiten Formwalze W2 kombiniert werden, die zwei Gruppen von Vertiefungen V1 und V2 aufweist. Mit dieser Anordnung lassen sich erfindungsgemäss unterschiedliche Dosierungsformen herstellen, die durch die Kombinationen V1 (W1) und V1 (W2) beziehungsweise V1 (W1) und V2 (W2) vorbestimmt sind.

Es kann es vorteilhaft sein, dass die Vertiefungen unterschiedlicher Gruppen auf getrennten Spuren auf der Formwalze angeordnet sind. Unter Spuren ist hier insbesondere eine Anordnung zu verstehen, bei der die Schwerpunkte der Vertiefungen einer Gruppe in Umlaufrichtung hintereinander auf der Walzenoberfläche angeordnet sind. Die einzelnen Spuren sind gegebenenfalls in axialer Richtung beabstandet zueinander angeordnet.

Diese Anordnung erlaubt es, dass man die verschiedenen Gruppen von erhaltenen Dosierungsformen nach der Formung bzw. nach hinreichendem Abkühlen nach Gruppen getrennt vom Formwerkzeug abnimmt, Man kann so die Dosierungsformen auf einfachem Wege nach Gruppen sortiert erhalten. Die nach Gruppen getrennte Abnahme der Dosierungsformen kann beispielsweise durch getrenntes Sammeln der Dosierungsformen jeder Gruppe in separaten Behältnissen erfolgen, gegebenfalls unter Zuhilfenahme von separaten Trichtern, Leitblechen, Absaugvorrichtungen und ähnlichen Einrichtungen für jede Gruppe, falls die Dosierungsformen einzeln aus den Vertiefungen austreten bzw. sich, beispielsweise mit den zuvor beschrieben Abstreifvorrichtungen, einzeln aus den Vertiefungen entnehmen lassen. Die nach Gruppen getrennte Abnahme gelingt besonders leicht, falls die Dosierungsformen als durch Verbindungsgrate oder Verbindungsstege verbundene Streifen oder Bänder erhalten werden. Vorteilhaft werden die Dosierungsformen anschliessend nach Gruppen getrennt vereinzelt, z.B. durch Zerschneiden der Bänder, und gegebenfalls nachbearbeitet, wie z.B. entgratet und/oder arrondiert, wie in der DE-A-196 29753 beschrieben, auf die hiermit Bezug genommen wird. Die nach Gruppen getrennte Abnahme der unterschiedlichen Dosierungsformen macht eine anschliessende Sortierung entbehrlich und ist insbesondere dann vorteilhaft, wenn sich die Dosierungsformen in Form und Volumen bzw. Gewicht so wenig unterscheiden, dass eine Trennung durch Siebung und/oder Gewichtsklassierung erschwert oder unmöglich ist.

Ebenfalls vorteilhaft ist die Anordnung der Vertiefungen einer Gruppe in Spuren, wenn man zusätzlich zu dem plastischen Gemisch als Folien vorliegende Materialien jeweils zwischen Schmelze und Formwalze beziehungsweise Formband bringt, wodurch gleichzeitig mit der Formung des plastischen Gemischs zu Dosierungsformen eine Umhüllung und/oder eine Verpackung der Dosierungsform erreicht werden kann, wie in der WO-96/19963 beschrieben, auf die hiermit Bezug genommen wird.

Alternativ werden die Dosierungsformen gesammelt und anschliessend nach Gruppen sortiert. In einer bevorzugten Ausführungsform erhält man nach Gruppen sortierte Dosierungsformen durch Siebung und/oder Gewichtsklassierung der erhaltenen Dosierungsformen. Dies ist insbesondere dann geeignet, wenn sich die unterschiedlichen Dosierungsformen hinreichend in Form und/oder Volumen bzw. Gewicht unterscheiden. Gegebenenfalls kann es notwendig sein, die erhaltenen Dosierungsformen nach der Abnahme vom Formwerkzeug erst zu vereinzeln, bevor ein Sortieren durch Siebung und/oder Gewichtsklassierung erfolgen kann. Dies ist insbesondere dann sinnvoll, wenn die Dosierungsformen beim Austritt aus dem Formwerkzeug noch ganz oder teilweise miteinander verbunden sind. Verfahren zum Sortieren von Dosierungsformen durch Siebung und/oder Gewichtsklassierung sind dem Fachmann bekannt.

Die erhaltenen unterschiedlichen Dosierungsformen können gegebenfalls gemeinsam oder nach Gruppen getrennt analog zu bekannten Verfahren, bei denen einheitliche Dosierungsformen erhalten wurden, nachbearbeitet, z.B. mit einem Überzug versehen, werden.

In der erfindungsgemäßen Vorrichtung werden zwei Formwalzen eingesetzt, deren Vertiefungen so gestaltet und angeordnet sind, dass die Formwalzen sowohl in einer ersten als auch in einer zweiten Orientierung vorliegen können, wobei die erste Orientierung durch Drehen einer der beiden Formwalzen um 180° bezüglich mindestens einer zur Längsachse dieser Formwalze senkrecht stehenden Achse in die zweite Orientierung überführt werden kann. Das heisst, eine der Formwalzen kann aus dem Formwerkzeug ausgebaut und so wieder eingebaut werden, dass das ursprünglich linke Ende nunmehr rechts zu liegen kommt; gegebenenfalls müssen die Walzen anschliessend neu synchronisiert werden. Die Kompatibilität der Vertiefungen in beiden Orientierungen ist z.B. gewährleistet, wenn die Umrisslinien der Vertiefungen auf einer Abrollung der Mantelflächen jeder Formwalze punktsymmetrisch zu mindestens einem Punkt angeordnet sind.

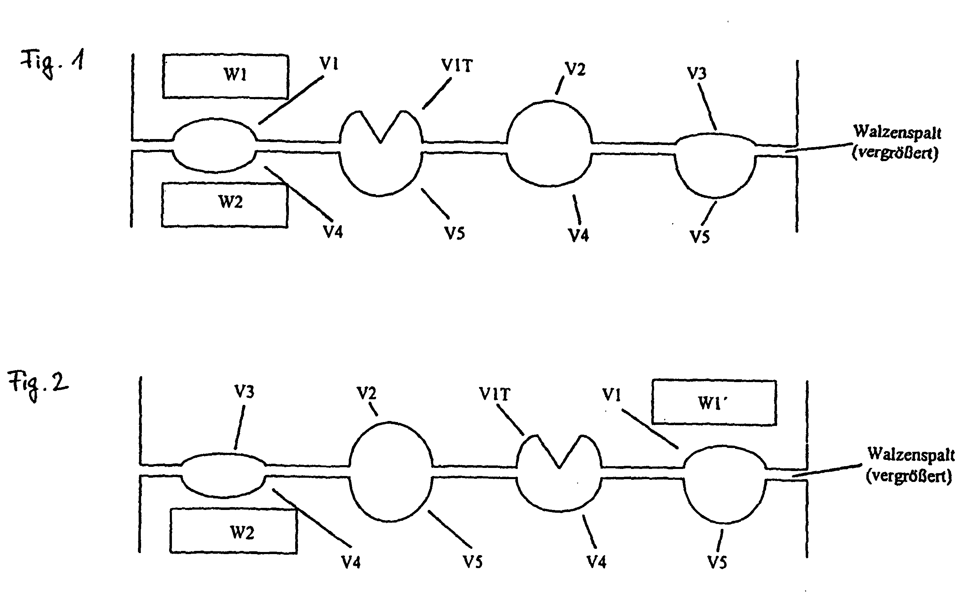

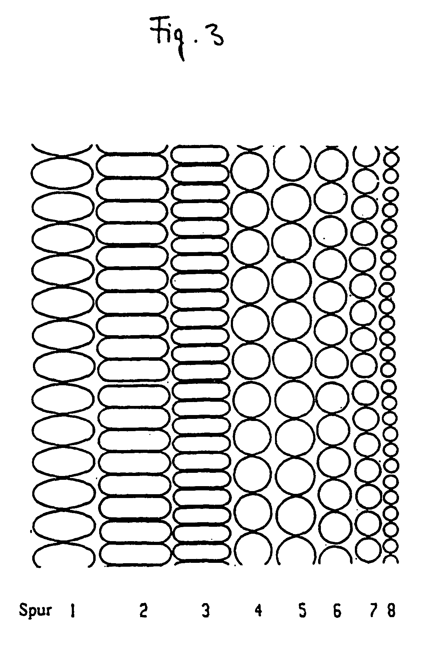

Die vorstehend beschriebene Anordnung der Umrisslinien ist insbesondere dann sinnvoll, wenn sich in der zweiten Orientierung andere Kombinationen von Vertiefungen ergeben als in der ersten Orientierung. Dies lässt sich an dem in der Fig. 1 und 2 gezeigten Fall veranschaulichen, bei dem ein Paar von gegenläufigen Formwalzen W1 und W2 verwendet wird, wobei die Formwalze W1 vier Spuren von Vertiefungen, V1, V1T, V2, V3 und die Formwalze W2 alternierende Spuren V4, V5, V4, V5 aufweist. Die Vertiefungen V1, V2, V3, V4 und V5 unterscheiden sich in Form und Volumen. V1 und V1T unterscheiden sich durch Anwesenheit eines Stegs in V1T, der zur Bildung einer Bruchrille und damit zu einer teilbaren Dosierungsform führt. Alle Vertiefungen auf W1 und W2 sollen gleiche Umrisslinien aufweisen und auf der jeweiligen Spur in gleicher Anzahl äquidistant in Umlaufrichtung hintereinander angeordnet sein. Die Abstände der Spuren untereinander und die Abstände der äusseren Spuren zu den Enden der Formwalze sind jeweils gleich gewählt. Die mit einer solchen Kombination von Formwalzen möglichen Dosierungsformen sind in den Tabellen 1 und 2 dargestellt. Dabei steht W1' für die wie zuvor beschrieben um 180° gedrehte Formwalze W1.

Diese Veranschaulichung demonstriert, dass sich so mit einer Vorrichtung eine grosse Zahl unterschiedlicher Dosierungsformen herstellen lässt.

**Tabelle 1**

| (erste Orientierung) | | | | |
|---|---|---|---|---|
| W1 | Spur 1 | Spur 2 | Spur 3 | Spur 4 |
| | V1 | V1T | V2 | V3 |
| W2 | Spur 1 | Spur 2 | Spur 3 | Spur 4 |
| | V4 | V5 | V4 | v5 |
| Resultierende Dosierungsform | V1 + V4 | V1T + V5 | V2 + V4 | V3 + V5 |

**Tabelle 2**

| (zweite Orientierung) | | | | |
|---|---|---|---|---|
| W1' (um 180° gedreht) | Spur 4 | Spur 3 | Spur 2 | Spur 1 |
| | V3 | V2 | V1T | V1 |
| W2 | Spur 1 | Spur 2 | Spur 3 | Spur 4 |
| | V4 | V5 | V4 | V5 |
| Resultierende Dosierungsform | V3 + V4 | V2 + V5 | V1T + V4 | V1 + V5 |

In jeder der beiden Orientierungen kommen die Umrisslinien der Vertiefungen der einen Formwalze und der Vertiefungen der anderen Formwalze bei einer gegenläufigen Rotation der Formwalzen im Spalt im Wesentlichen zur Deckung. Die Drehachse für die Drehung um 180° ist durch einen Punkt auf der Walzenoberfläche, der auf der Abrollung der Mantelfläche der Symmetriepunkt ist, und seine lotrechte Projektion auf die Walzenlängsachse festgelegt. Für den Fall geringster Symmetrie (einfache Punktsymmetrie) gibt es nur eine Drehachse, die wie vorstehend beschrieben definiert ist. Wenn es mehrere Symmetriepunkte auf den Abrollungen der Mantelflächen der Formwalzen gibt, gibt es genauso viel Drehachsen, die durch die lotrechte Projektion dieser Symmetriepunkte auf die Walzenlängsachse gegeben sind.

Im einfachsten Fall weisen alle Vertiefungen gleiche Umrisslinien, z.B. runde Umrisslinien, auf und die Anordnung der Vertiefungen beider Formwalzen ist bezüglich einer zu der Walzenachse senkrechten Mittelebene spiegelsymmetrisch.

Das nachfolgende Beispiel soll die Erfindung erläutern ohne sie einzuschränken.

### Beispiel (nicht erfindungsgemaß)

In einem gleichsinnig drehenden Doppelschneckenextruder (ZSK-40 der Firma Werner & Pfleiderer, Stuttgart) wurde ein plastisches wirkstoffhaltiges Gemisch, das als Wirkstoff 48,0 Gew.-% Verapamil-Hydrochlorid und als thermoplastisches physiologisch verträgliches polymeres Bindemittel ein Gemisch aus 31,5 Gew.-% Hydroxypropylcellulose der Fa. Aqualon und 17,5 Gew.-% Hydroxypropylmethylcellulose der Fa. Colorcon und als Hilfsstoff 3,0 Gew.-% Lecithin enthielt, erzeugt und das wirkstoffhaltige plastische Gemisch mit einem Durchsatz von 15 kg/h extrudiert. Die Temperatur des plastischen Gemischs wurde kurz vor der Austrittsdüse gemessen und betrug 120°C. Der Austrag erfolgte über eine 12 cm breite Breitschlitzdüse. Das ausgetragene Gut wurde in einen Kalander mit zwei gegenläufig rotierenden Formwalzen eingeführt. Jede der Formwalzen wies 8 Gruppen von Vertiefungen auf, die in 8 Spuren (Spuren 1 bis 8) angeordnet sind, wie in Figur 3 abgebildet. Die Spuren 1 bis 8 sind von links nach rechts angeordnet. Die Vertiefungen einer Gruppe auf der einen Formwalze kommen bei der Rotation mit den Vertiefungen der entsprechenden Gruppe auf der anderen Formwalze im Spalt zur Deckung. Die Umrisslinien der Gruppe 1 (Spur 1) waren elliptisch, die der Gruppe 2 (Spur 2) und Gruppe 3 (Spur 3) länglich und die der Gruppen 4 bis 8 (Spuren 4 bis 8) rund. Ein Ausschnitt aus der Abrollung der Mantelfläche einer der beiden Formwalzen ist in Figur 3 oben abgebildet.

Die durch Kalandrierung hergestellten unterschiedlichen Dosierungsformen wurden als zusammenhängendes Band von dem Kalander abgenommen und kühlten auf einem nachgeschalteten Transportband ab. Anschliessend wurden die Dosierungsformen vereinzelt und durch Gewichtsklassierung nach Gruppen 1 bis 8 getrennt. Das Gewicht und der Wirkstoffgehalt der nach dem erfindungsgemässen Verfahren mit der erfindungsgemässen Vorrichtung hergestellten unterschiedlichen Dosierungsformen ist in Tabelle 3 dargestellt. Das angegebene Gewicht bzw. der angegebene Wirkstoffgehalt der Dosierungsformen der unterschiedlichen Spuren stellt das Gewichtsmittel aus jeweils 100 Dosierungsformen einer Gruppe dar.

**Tabelle 3**

| Formwalze 1 Formwalze 2 | Form der umrisslinie | Form der Dosierungsform | Gewicht¹⁾ der Dosierungsform [mg] | Wirkstoffgehalt¹⁾ der Dosierungsform [mg] |
|---|---|---|---|---|
| Spur 1 (Gruppe 1) | elliptisch | mandelförmig | 408 | 196 |
| Spur 2 (Gruppe 2) | länglich | stäbchenförmig | 656 | 315 |
| Spur 3 (Gruppe 3) | länglich | stäbchenförmig | 325 | 156 |
| Spur 4 (Gruppe 4) | rund | linsenförmig | 388 | 186 |
| Spur 5 (Gruppe 5) | rund | linsenförmig | 294 | 141 |
| Spur 6 (Gruppe 6) | rund | linsenförmig | 160 | 77 |
| Spur 7 (Gruppe 7) | rund | linsenförmig | 81 | 39 |
| Spur 8 (Gruppe 8) | rund | linsenförmig | 22 | 11 |

| | | | | |
|---|---|---|---|---|
| 1) Gewichtsmittel aus je 100 Dosierungsformen einer Gruppe | | | | |

## Patentansprüche

1. Vorrichtung zur Herstellung , unterschiedlicher Dosierungsformen, umfassend zwei Formwalzen (W1, W1', W2), die zur Formung eines plastischen Gemischs zusammenwirken, wobei
a) die Formwalzen eine Vielzahl von Vertiefungen (V1, V1T, V2, V3, V4, V5) zur Aufnahme und Formung des plastischen Gemischs aufweisen,
b) die Formwalzen gegenläufig rotierbar sind und die Umrisslinien der Vertiefungen der einen Formwalze mit den Umrisslinien der Vertiefungen der anderen Formwalze bei der Rotation im Spalt zwischen den Formwalzen paarweise im Wesentlichen zur Deckung kommen und wobei
c) wenigstens eine Formwalze mindestens zwei sich im Volumen und/oder der Form unterscheidende Gruppen von Vertiefungen aufweist,
**dadurch gekennzeichnet, dass** die Vertiefungen so gestaltet und auf den Walzenoberflächen angeordnet sind, dass die Formwalzen sowohl in einer ersten (W1) als auch in einer zweiten (W1') Orientierung vorliegen können, wobei die erste Orientierung durch Drehen einer der beiden Formwalzen um 180° bezüglich mindestens einer zur Längsachse dieser Formwalze senkrecht stehenden Achse in die zweite Orientierung überführt werden kann und umgekehrt.

2. Verfahren zur Herstellung fester Dosierungsformen durch Formung eines plastischen wirkstoffhaltigen Gemischs unter Verwendung der Vorrichtung nach Anspruch 1.

3. Verfahren nach Anspruch 2, **dadurch gekennzeichnet, dass** die Vertiefungen unterschiedlicher Gruppen auf getrennten Spuren auf der Formwalze angeordnet sind.

4. Verfahren nach Anspruch 3, **dadurch gekennzeichnet, dass** man die erhaltenen Dosierungsformen nach Gruppen getrennt vom Formwerkzeug abnimmt.

5. Verfahren nach Anspruch 2 oder 3, **dadurch gekennzeichnet, dass** man die erhaltenen Dosierungsformen durch Siebung und/oder Gewichtsklassierung nach Gruppen sortiert.

## Claims

1. Apparatus for producing various dosage forms comprising two moulding rolls (W1, W1', W2) which cooperate to mould a plastic mixture, with
a) the moulding rolls having a plurality of depressions (V1, V1T, V2, V3, V4, V5) to receive and mould the plastic mixture,
b) the moulding rolls being able to rotate in opposite directions, and the outlines of the depressions in one moulding roll essentially coinciding pairwise with the outlines of the depressions in the other moulding roll during rotation in the gap between the moulding rolls,
c) at least one moulding roll having at least two groups, which differ in volume and/or shape, of depressions, **characterized in that** the depressions are shaped and arranged on the surfaces of the rolls so that both a first (W1) and a second (W1') orientation of the moulding rolls is possible, it being possible to convert the first orientation into the second orientation by rotating one of the two moulding rolls by 180° relative to at least one axis perpendicular to the long axis of this moulding roll, and vice versa.

2. Process for producing solid dosage forms by moulding a plastic mixture containing an active ingredient using the apparatus according to Claim 1.

3. Process according to Claim 2, **characterized in that** the depressions in different groups are arranged on separate lanes on the moulding roll.

4. Process according to Claim 3, **characterized in that** the resulting dosage forms are removed in separate groups from the moulding tool.

5. Process according to Claim 2 or 3, **characterized in that** the resulting dosage forms are sorted into groups by sieving and/or classifying by weight.

## Revendications

1. Dispositif de fabrication de présentations pharmaceutiques différentes qui comprend deux cylindres de moulage (W1, W1', W2) agissant conjointement pour mouler un mélange plastique, dans lequel
a) les cylindres de moulage comportent une pluralité de cavités (V1, V1T, V2, V3, V4, V5) destinées à recevoir et à mouler un mélange plastique,
b) les cylindres de moulage peuvent être mis en rotation opposée et, lors de la rotation, les lignes de contour des cavités de l'un des cylindres se superposent pour l'essentiel et par paires, au niveau de l'interstice entre les cylindres, aux lignes de contour des cavités de l'autre cylindre
c) et dans lequel au moins un des cylindres comporte au minimum deux groupes de cavités de formes et/ou de volumes différents,
**caractérisé en ce que** les cavités sont configurées et disposées à la surface des cylindres de manière telle que les cylindres peuvent se présenter selon une première orientation (W1) ou bien selon une deuxième orientation (W1'), le changement de la première vers la deuxième orientation, et inversement, étant possible en tournant un des deux cylindres de 180 degrés par rapport à au moins un axe perpendiculaire à l'axe longitudinal de ce cylindre.

2. Procédé de fabrication de présentations pharmaceutiques différentes en forme solide, par moulage d'un mélange plastique contenant des additifs et en utilisant le dispositif selon la revendication 1.

3. Procédé selon la revendication 2, **caractérisé en ce que** les cavités de groupes différents sont agencées sur des voies séparées sur le cylindre de moulage.

4. Procédé selon la revendication 3, **caractérisé en ce que** les présentations pharmaceutiques différentes, sont retirées du dispositif de moulage séparément suivant leur groupe.

5. Procédé selon la revendication 2 ou 3, **caractérisé en ce que** les présentations pharmaceutiques différentes obtenues, sont triées par groupes, par tamisage et/ou classification pondérale.
